# EUROPEAN PATENT APPLICATION

(11) **EP 2 888 988 A1**
(43) Date of publication of application: **01.07.2015**
(21) Application number: 13831452.1
(22) Date of filing: 21.08.2013
(51) Int. Cl.: A61B 1/00, A61B 10/00, G01N 21/64

(54) **PHOTODYNAMIC DIAGNOSIS APPARATUS PROVIDED WITH COLLIMATOR**

(30) Priority: 23.08.2012 JP 2012184037
(71) Applicant: SBI Pharmaceuticals Co., Ltd., Tokyo 106-6020 (JP)
(72) Inventor: INOUE, Katsushi, Tokyo 106-6020 (JP); HIRAMITSU, Masanori, Tokyo 106-6020 (JP); NAKAJIMA, Motowo, Tokyo 106-6020 (JP); TANAKA, Tohru, Tokyo 106-6020 (JP)
(74) Representative: Duckett, Anthony Joseph
(86) International application number: PCT/JP2013/004933
(87) International publication number: WO 2014/030344

(57) **Abstract**

An object of the present invention is to provide a PDD diagnosis apparatus that even if there is a distance from an irradiation portion of an optical fiber to a diseased tissue, enables provision of a fluorescence detection sensitivity equivalent to that in a case where irradiation is provided in such a manner that the irradiation portion of the optical fiber is brought close to the diseased tissue. A diagnosis apparatus including: a light source unit that emits excitation light to a photosensitizing substance accumulated in a diseased tissue; a detection unit that detects fluorescence produced from the photosensitizing substance; and an excitation light irradiation/fluorescence detection optical fiber including an output/input end with a collimator connected thereto, in which the excitation light emitted from the light source unit is guided to the excitation light irradiation/fluorescence detection optical fiber, and the excitation light output from the output/input end is provided in such a manner that the excitation light is collimated by the collimator and excites the photosensitizing substance accumulated in the diseased tissue; and the fluorescence produced by the photosensitizing substance excited by the excitation light is input from the output/input end via the collimator, and the input fluorescence is guided to the excitation light irradiation/fluorescence detection optical fiber and the fluorescence produced from the photosensitizing substance is detected in the detection unit is used.

## Description

### Technical Field

The present invention relates to a photodynamic diagnosis apparatus provided with a collimator, and more specifically relates to a diagnosis apparatus for identifying a diseased tissue (focal site), the apparatus including, e.g., a light source unit that emits excitation light, a detection unit that detects fluorescence produced from a photosensitizing substance, and an excitation light irradiation/fluorescence detection optical fiber including an output/input end with a collimator connected thereto.

### Background Art

Cancer (malignant tumor) is the greatest killer in Japan, and one out of every two Japanese persons is said to be affected by cancer. In the other developed countries, cancer is also high in the list of death causes. Therefore, development of effective diagnostic methods and therapeutic methods for cancers is the earnest wish of people of the world including Japanese people.

As a cancer diagnostic method, photodynamic diagnosis (hereinafter referred to also as "PDD") has been developed. PDD is a diagnostic method in which a photosensitizing substance is accumulated in a diseased tissue, irradiated with excitation light to produce fluorescence and the fluorescence is observed and detected to determine whether or not there is a disease and identify a diseased tissue. For example, 5-aminolevulinic acid (hereinafter also referred to as "5-ALA") is known as an intermediate of a pigment biosynthesis pathway, which is widely present in animals, plants and fungi, and is normally biosynthesized from succinyl CoA and glycine using 5-ALA synthetase. It is known that although 5-ALA itself has no light sensitivity, 5-ALA is metabolized into protoporphyrin IX (PpIX) in cells by a group of enzymes in a heme biosynthetic pathway and accumulated in a tumor tissue and/or new blood vessels. It is known that upon receipt of excitation light of around 410 nm, PpIX produces red fluorescence having a peak at around 636 nm and thus is used for diagnosis of a tumor by means of PDD.

As stated above, intraoperative fluorescence diagnosis in which a malignant tumor is removed while observing red fluorescence produced by a fluorescent substance PpIX by exciting PpIX specifically accumulated in the malignant tumor after administration of 5-ALA by means of violet light is applied in various types of tumorectomy; however, no sufficient fluorescence intensity can be obtained in brain tumor removal surgery in which an operation is performed using a surgical microscope and tumor removal performed endoscopically, resulting in a limitation on visual fluorescence diagnosis. Also, since malignant tumors have the property of growing by invading normal tissues, white autofluorescence produced by the normal tissues makes it difficult to visually recognize red light for which humans have a low spectral sensitivity. The present inventors have developed an apparatus in which violet semiconductor laser light is guided close to a diseased tissue via an irradiation fiber to provide spot irradiation and a fluorescence spectrum is concurrently detected via a measurement fiber, which is paired with the irradiation fiber, to detect a fluorescence spectrum peak of a wavelength of 636 nm, which is specific to PpIX, thereby notifying a surgeon of localization of PpIX, that is, localization of a tumor by means of a detection sound (Patent document 1).

Also, the present inventors have proposed a 5-ALA-used cervical cancer determination/therapy (ALA-PDD/PDT) system sequentially including the steps: a step of orally administering any of 5-ALAs; an ALA-PDD step of irradiating a uterine cervix with excitation light having wavelengths of 380 to 420 nm to detect red fluorescence, thereby specifying a PpIX-accumulated site and determining a range of a lesion tissue to be resected; an ALA-photodynamic therapy (hereinafter also referred to as "PDT") step of providing irradiation with light having wavelengths of 625 to 642 nm to necrotize the lesion tissue (Patent document 2).

On the other hand, a fluorescence diagnosis apparatus for observing a focal site based on fluorescence from a photosensitive substance accumulated in the focal site, the apparatus including: an excitation light source that emits excitation light having a wavelength peak value that is shorter than a center wavelength of the fluorescence from the photosensitive substance, a difference between the peak value and the center wavelength of the fluorescence from the photosensitive substance exceeding 12 nm and being no more than 16 nm; a collimator lens that collimates the excitation light; excitation light wavelength shaping means having a center wavelength of a band-pass filter that corresponds to or is close to the peak value of the excitation light, the excitation light wavelength shaping means receiving the excitation light passing from the collimator lens toward the focal site; light isolation means having a center wavelength of a band-stop filter that corresponds to or is close to the peak value of the excitation light, the light isolation means receiving the fluorescence from the photosensitive substance excited by the excitation light and reflection of the excitation light from the focal site; and an image pickup device that picks up an image of fluorescence information obtained by the light isolation means (Patent document 3) is known.

Also, a laser apparatus including: a laser light source; an external resonator including an input coupling element; a photodetection element; and a collimator lens for aligning an optical axis of laser light from the laser light source with the external resonator based on a light output detected by the photodetection element, in which a particular position between the collimator lens disposed on the light source side and the input coupling element in the external resonator and a light receiving surface of the photodetection element are arranged so as to form a geometric optical conjugate relationship (Patent document 4) is also known.

In addition, an irradiation source for use in photodynamic therapy, the irradiation source including a two-dimensional array of LEDs (light-emitting diodes) and further including means for collimating light emitted from the LEDs (Patent document 5) has also been proposed.

### Prior Art Documents

### Patent documents

Patent document 1: Japanese Unexamined Patent Application Publication No. 2010-240078
Patent document 2: Japanese Unexamined Patent Application Publication No. 2011-001307
Patent document 3: Japanese Unexamined Patent Application Publication No. 2007-303990
Patent document 4: Japanese Unexamined Patent Application Publication No. 2007-317871
Patent document 5: Japanese Unexamined Patent Application Publication (Translation of PCT Application) No. 2004-528930

### Summary of the Invention

### Object to be Solved by the Invention

In conventional PDD apparatuses, excitation light provided from an end of an optical fiber has a diffusion angle of approximately 20°, and thus, in order to obtain a sufficient excitation intensity, it is necessary to provide irradiation close to a diseased tissue (focal site), and since a core diameter of a fluorescence receiving surface is small, that is, 300 to 400 µm, as the distance from an irradiation portion is longer, the amount of fluorescence received decreases and the sensitivity thus decreases, and therefore it is necessary to provide irradiation close to a diseased tissue. Thus, during irradiation with violet laser, if a distal end of an optical fiber comes into contact with a tissue, the distal end largely absorbs, in particular, hemoglobin in the blood, which sticks to or is carbonized on an end face, which may result in failure to output excitation light, and in each of such cases, a surgeon needs to clean the distal end with an alcohol swab. Also, in intraoperative fluorescence diagnosis for brain tumor, which is widely used in clinical studies, an operation is performed with a suction tube in one hand and a bipolar or ultrasound surgical knife or the like in the other hand, and thus, if it is necessary to hold a handpiece probe with an optical fiber for fluorescence diagnosis incorporated therein, the operation may fairly be complicated.

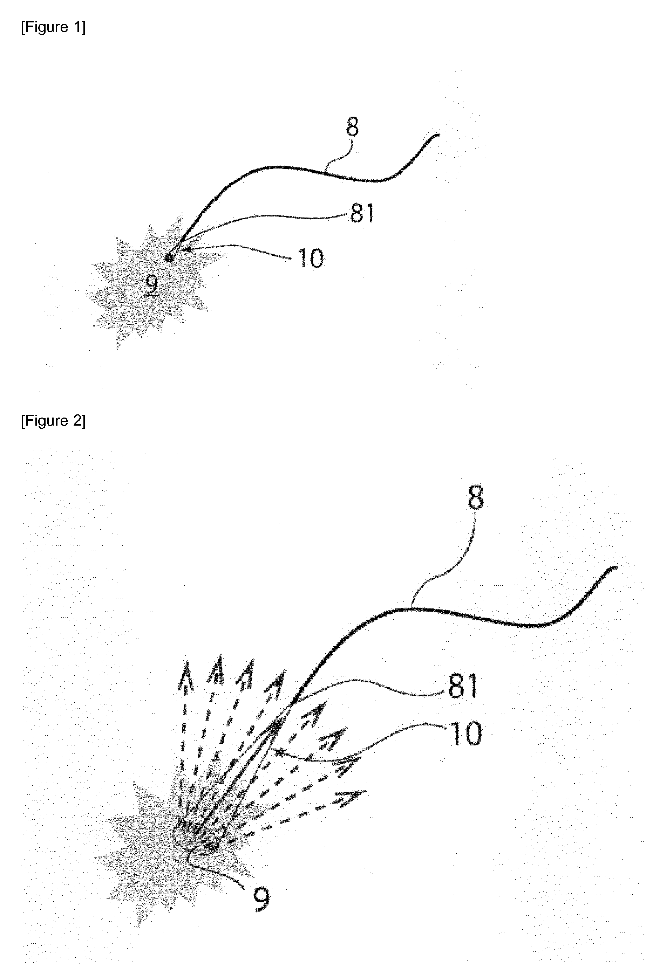

As illustrated in Figure 1, where 5-ALA-induced PpIX accumulated in a diseased tissue (focal site) 9 is excited by violet laser light output from an excitation light irradiation/fluorescence detection optical fiber 8 as is conventionally done, it is necessary to provide irradiation with an optical fiber distal end 81 brought close to the diseased tissue 9 in order to excite the diseased tissue 9 at a sufficient excitation intensity because a diffusion angle from the optical fiber distal end 81 is around 20°, and during irradiation with excitation light 10, if a tissue adheres to the optical fiber distal end 81, the optical fiber distal end 81 largely absorbs, in particular, hemoglobin, which thus sticks to or is carbonized in the optical fiber distal end in a short period of time, resulting in the problem of failure in irradiation with the excitation light 10.

Also, as illustrated in Figure 2, if a diseased tissue 9 and an optical fiber distal end 81 are used with a distance from each other, an irradiation diameter becomes large and thus no sufficient excitation intensity can be obtained, resulting in the problem of not only weakening of fluorescence emission but also substantial decrease in amount of fluorescence that can be received by a light receiving surface with a core diameter of 300 to 400 µm, causing fluorescence detection sensitivity decrease.

An object of the present invention is to provide a PDD diagnosis apparatus that even if there is a distance from an irradiation portion of an optical fiber to a diseased tissue, enables provision of a fluorescence detection sensitivity that is equivalent to that in a case where a diseased tissue is irradiated with the irradiation portion of the optical fiber brought close to the diseased tissue, that is, a PDD diagnosis apparatus that can excite a photosensitizing substance accumulated in a diseased tissue at a sufficient excitation intensity, accurately receive fluorescence produced from the photosensitizing substance, and can accurately identify, for example, a peripheral part of a tumor part or a low-invasion site around the tumor part, which is difficult to observe by the naked eye.

### Means to Solve the Object

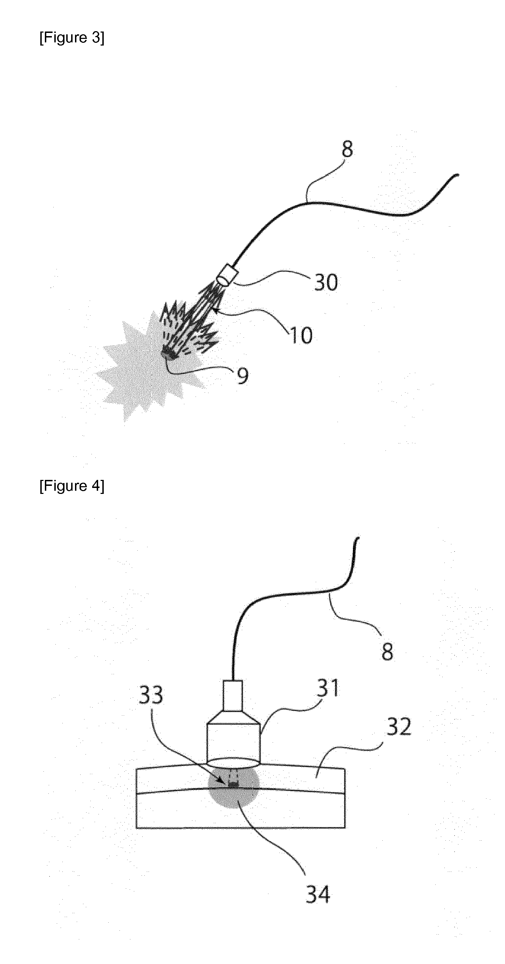

The present inventors first attached a transparent tube to an optical fiber distal end in order to prevent the optical fiber distal end from adhering to a tissue during surgery. However, it turned out that a distance from the optical fiber distal end to a diseased tissue becomes long, resulting in not only failure in excitation at a sufficient excitation intensity but also failure in accurate reception of fluorescence produced from a photosensitizing substance, causing problems in terms of sensitivity. Therefore, as illustrated in Figure 3, a collimator 30 is attached to a distal end of an excitation light irradiation/fluorescence detection optical fiber 8 to provide excitation light 10 collimated by the excitation light irradiation/fluorescence detection optical fiber 8, enabling a diseased tissue 9 to be irradiated at a relatively high intensity from a distant position. Concurrently, fluorescence in an irradiation portion is efficiently guided to the excitation light irradiation/fluorescence detection optical fiber according to the principle of telescope, enabling provision of a fluorescence detection sensitivity equivalent to that in a case where irradiation is provided with the optical fiber brought close to the diseased tissue. As a result, it has become possible to prevent pollution of an optical fiber end face due to adherence of body fluid to the optical fiber end face, which is caused when irradiation is provided with the optical fiber brought close to a diseased tissue, and excessive irradiation of a diseased tissue with excitation light having a high intensity. Also, only fluorescence and reflection light in the irradiation portion can be detected according to the principle of telescope in the collimator at the optical fiber distal end, enabling substantial reduction of the influence of external light such as illuminating light on the fluorescence detection. Furthermore, as a result that an excitation light irradiation/fluorescence detection optical fiber with a collimator attached to a distal end thereof is attached to the vicinity of an objective lens of a surgical microscope used in brain tumor removal, excitation/fluorescence detection of one point in the field of view in conjunction with the surgical microscope is enabled, and thus, the burden of providing irradiation with a handpiece, which includes an excitation light irradiation/fluorescence detection optical fiber incorporated therein, brought close to a diseased tissue during surgery can be removed.

In other words, the present invention relates to (1) a diagnosis apparatus for identifying a diseased tissue by irradiating a photosensitizing substance accumulated in the diseased tissue with excitation light and detecting fluorescence generated from the photosensitizing substance, the apparatus including: a light source unit that emits the excitation light; a detection unit that detects the fluorescence generated from the photosensitizing substance; and an excitation light irradiation/fluorescence detection optical fiber including an output/input end with a collimator connected thereto, in which the excitation light emitted from the light source unit is guided to the excitation light irradiation/fluorescence detection optical fiber, and the excitation light output from the output/input end is provided in such a manner that the excitation light is collimated by the collimator and excites the photosensitizing substance accumulated in the diseased tissue; and the fluorescence generated by the photosensitizing substance excited by the excitation light is input from the output/input end via the collimator, and the input fluorescence is guided to the excitation light irradiation/fluorescence detection optical fiber and the fluorescence generated from the photosensitizing substance is detected in the detection unit.

Also, the present invention relates to: (2) the diagnosis apparatus according to (1) above in which the diseased tissue is a peripheral part of a tumor part, and/or a low-invasion site around the tumor part, the low-invasion site being difficult to observe by the naked eye; (3) the diagnosis apparatus according to (1) or (2) above in which the photosensitizing substance is protoporphyrin IX (PpIX); (4) the diagnosis apparatus according to any one of (1) to (3) above in which the excitation light irradiation/fluorescence detection optical fiber has a core diameter of 300 to 400 µm; (5) the diagnosis apparatus according to any one of (1) to (4) above in which the excitation light irradiation/fluorescence detection optical fiber is incorporated in a center of a bundle fiber light guide that guides violet light; (6) the diagnosis apparatus according to any one of (1) to (5) above, further including a light output optical fiber optically connected to the light source unit at a light emission end of the light source unit, the light output optical fiber guiding the excitation light emitted from the light source unit to a reflection port in a light mixing/isolating three-port module, a band reflection/transmission mirror in the light mixing/isolating three-port module, the band reflection/transmission mirror reflecting the excitation light output from the reflection port, outputting the excitation light to a common port that is in communication with the excitation light irradiation/fluorescence detection optical fiber and transmitting the fluorescence input from the excitation light irradiation/fluorescence detection optical fiber to the common port, and a light input optical fiber optically connected to the detection unit at a light entrance end of the detection unit, the light input optical fiber guiding the fluorescence passing through the band reflection/transmission mirror to the detection unit via a transmission port in the light mixing/isolating three-port module; (7) the diagnosis apparatus according to any one of (1) to (6) above, further including a means for dispersing the fluorescence, and further including an alarm sounding means for, if a detected amount of the fluorescence generated from the photosensitizing substance is equal to or exceeds a set value, outputting a detection sound for notification; and (8) the diagnosis apparatus according to any one of (1) to (7) above in which a transparent tube or a transparent rod is further joined to a distal end of the collimator.

Furthermore, the present invention relates to (9) an intraoperative diagnosis system comprising a combination of a diagnosis apparatus according to any one of (1) to (8) above and a surgical microscope having a fluorescence diagnosis mode, in which the excitation light irradiation/fluorescence detection optical fiber with the collimator connected thereto in the diagnosis apparatus is incorporated in the surgical microscope as a probe.

### Effect of the Invention

A diagnosis apparatus according to the present invention including an excitation light irradiation/fluorescence detection optical fiber with a collimator connected to an output/input end thereof enables efficient light collection by making not only excitation light but also fluorescence produced by an excited photosensitizing substance pass through the collimator. As a result, even if there is a distance from an irradiation portion of the optical fiber to a diseased tissue, a fluorescence detection sensitivity equivalent to that in a case where irradiation is provided with the irradiation portion of the optical fiber brought close to the diseased tissue can be obtained. For example, use of a surgical microscope having a fluorescence diagnosis mode, in which an excitation light irradiation/fluorescence detection optical fiber with a collimator connected thereto is incorporated as a probe, enables identification of, in particular, a low-invasion site around a tumor part that is difficult to observe by the naked eye, detection of which has conventionally been considered difficult. Furthermore, with a technique that is similar to the above, also, an excitation light irradiation/fluorescence detection optical fiber with a collimator attached to a distal end thereof is incorporated in any of various types of endoscopes such as flexible endoscopes including a digestive endoscope and a bronchial endoscope, and a laparoscope and a cystoscope, enabling the diagnosis apparatus according to the present invention to be applied in fluorescence diagnosis of a deep part or a narrow part distant from an endoscope distal end, which is difficult for an endoscope to enter.

### Brief Description of Drawings

[Figure 1] Figure 1 is a diagram illustrating a conventional technique (close irradiation). A fiber distal end is brought close to a tissue and the tissue is irradiated with excitation light and red fluorescence is detected concurrently.
[Figure 2] Figure 2 is a diagram illustrating a conventional technique (distant irradiation). Since an irradiation angle (approximately 20°) from a fiber is large, an irradiation diameter is large and an irradiation power intensity thus decreases, resulting in decrease in fluorescence intensity. Also, since fluorescence diffuses, the amount of fluorescence that can be received by a fiber end face (having a core diameter of less than 1 mm) is small, resulting in substantial decrease in detection sensitivity.
[Figure 3] Figure 3 is a diagram illustrating a technique according to the present invention in which a collimator is attached to an optical fiber distal end. Excitation light is collimated and provided to a diseased tissue with a relatively-small irradiation diameter, which results in increase in excitation intensity, and produced fluorescence is received by an irradiation portion in a size of an aperture of a lens (several millimeters to several tens of millimeters), which enables detection of fluorescence at a relatively-high sensitivity.
[Figure 4] Figure 4 is a schematic diagram of fluorescence diagnosis for lymph node metastasis.
[Figure 5] Figure 5 is a diagram illustrating an excitation light irradiation/fluorescence detection optical fiber with a small-aperture collimator attached thereto, which is employed in, e.g., an endoscope.
[Figure 6] Figure 6 is a diagram illustrating a hybrid fiber in which a fiber that guides violet laser light is incorporated in the center of a bundle fiber light guide that guides light from, e.g., a lamp light source configured to emit violet light or a violet LED light source.
[Figure 7] Figure 7 is a schematic diagram of a diagnosis apparatus according to the present invention, the diagnosis apparatus including a light mixing/isolating three-port module.
[Figure 8] Figure 8 is a schematic diagram of a surgical microscope having a fluorescence diagnosis mode.
[Figure 9] Figure 9 is a schematic diagram of a surgical microscope according to the present invention having a fluorescence diagnosis mode, in which a fiber probe with a collimator attached to a distal end thereof is incorporated.
[Figure 10] Figure 10 is a vertical cross-sectional view of a surgical microscope with an excitation light irradiation/fluorescence detection optical fiber externally attached thereto, a collimator being connected to the excitation light irradiation/fluorescence detection optical fiber.

### Mode of Carrying Out the Invention

For a diagnosis apparatus for identifying a diseased tissue according to the present invention, there is no specific limitations as long as the diagnosis apparatus is a diagnosis apparatus for identifying a diseased tissue of a subject by irradiating a photosensitizing substance accumulated in the diseased tissue with excitation light and detecting fluorescence produced from the photosensitizing substance, the apparatus including at least: a light source unit that emits the excitation light; a detection unit that detects the fluorescence produced from the photosensitizing substance; and an excitation light irradiation/fluorescence detection optical fiber including an output/input end with a collimator connected thereto, in which the excitation light emitted from the light source unit is guided to the excitation light irradiation/fluorescence detection optical fiber, the excitation light output from the output/input end is provided in such a manner that the excitation light is collimated by the collimator, and excites the photosensitizing substance accumulated in the diseased tissue; and in which the fluorescence produced by the photosensitizing substance excited by the excitation light is input from the output/input end via the collimator, and the input fluorescence is guided to the excitation light irradiation/fluorescence detection optical fiber and the fluorescence produced from the photosensitizing substance is detected in the detection unit, and examples of the subject can include mammals such as a human, a monkey, a dog and a cat.

For the diseased tissue, there are no specific limitations as long as the diseased tissue is a diseased tissue that allows a photosensitizing substance to be accumulated, and examples of the diseased tissue can include a tumor tissue, a lymph node metastasis, peritoneal dissemination, mesothelioma, normal thyroid localization and an inflamed site caused by allergic rhinitis. Furthermore, in taking an advantage of the diagnosis apparatus according to the present invention, that is, being able to identify localization of a diseased tissue, examples of the diseased tissue can include peripheral parts of a diseased site such as a local part, a tumor part and a peripheral part of the diseased tissue and a site around the disease such as a low-invasion site around a tumor part, which is difficult to observe by the naked eye.

Examples of the tumor can include cancers and tumors arising from, e.g., epithelial cells becoming malignant such as skin cancer, lung cancer, trachea/bronchus cancer, oral epithelial cancer, esophageal cancer, stomach cancer, colon cancer, rectum cancer, bowel cancer, liver/intrahepatic bile duct cancer, kidney cancer, pancreas cancer, prostate cancer, breast cancer, uterine cancer, ovarian cancer, bladder cancer and a brain tumor.

For the photosensitizing substance, any of various substances including an amino-acid derivative, an azo dye, a xanthene derivative, chlorin, a tetrapyrrole derivative and phthalocyanine, which are conventionally known as photosensitizing substances, can be used, and in particular, porphyrin-based photosensitizing substances such as protoporphyrin, hematoporphyrin, a hematoporphyrin derivative, benzoporphyrin and pentacarboxyporphyrin can be indicated as preferable examples, and among them, PpIX can be indicated as a particularly preferable example. In order to accumulate PpIX in a diseased tissue, any of compounds represented by general formula (I), R¹HNCH₂COCH₂CH₂COR² (in which R¹ represents a hydrogen atom or an acyl group, R² represents a hydrogen atom, a linear or branched alkyl group, a cycloalkyl group, an aryl group or an aralkyl group) and salts thereof (hereinafter may collectively be referred to as "ALAs") can be administered. The ALAs are metabolized quickly, i.e., in 24 hours, and have extremely few side effects such as photosensitivity and the ALAs are thus most suitable in PDD and PDT.

Among the above-indicated ALAs, a 5-ALA and a salt thereof in which R¹ and R² in formula (I) each represent a hydrogen atom can be indicated as preferable examples. 5-ALA is one type of amino acids called δ-aminolevulinic acid. Also, examples of a 5-ALA derivative can include compounds, other than the 5-ALAs, in which R¹ in formula (I) represents a hydrogen atom or an acyl group, R² in formula (I) represents a hydrogen atom, a linear or branched alkyl group, a cycloalkyl group, an aryl group or an aralkyl group.

Examples of the acyl group in formula (I) can include linear or branched alkanoyl groups having 1 to 8 carbon atoms such as formyl, acetyl, propionyl, butyryl, isobutyryl, valeryl, isovaleryl, pivaloyl, hexanoyl, octanoyl and benzylcarbonyl groups and aroyl groups having 7 to 14 carbon atoms such as benzoyl, 1-naphthoyl and 2-naphthoyl groups.

Examples of the alkyl group in formula (I) can include linear or branched alkyl groups having 1 to 8 carbon atoms such as methyl, ethyl, propyl, isopropyl, butyl, isobutyl, sec-butyl, tert-butyl, pentyl, isopentyl, neopentyl, hexyl, heptyl and octyl groups.

Examples of the cycloalkyl group in formula (I) includes cycloalkyl groups having 3 to 8 carbon atoms in which a saturated or partially unsaturated bond may be present such as cyclopropyl, cyclobutyl, cyclopentyl, cyclohexyl, cycloheptyl, cyclooctyl, cyclododecyl and 1-cyclohexenyl groups.

Examples of the aryl group in formula (I) can include aryl groups having 6 to 14 carbon atoms such as phenyl, naphthyl, anthryl and phenanthryl groups.

For the aralkyl group in formula (I), examples of the aryl moiety can include those the same as those of the aryl group indicated above, and examples of the alkyl moiety can include those the same as those of the alkyl group indicated above, and specific examples of the aralkyl group can include aralkyl groups having 7 to 15 carbon atoms such as benzyl, phenethyl, phenylpropyl, phenylbutyl, benzhydryl, trityl, naphthylmethyl and naphthylethyl groups.

For the 5-ALA derivative, a compound in which R¹ represents, e.g., a formyl, acetyl, propionyl or butyryl group or a compound in which R² represents a methyl, ethyl, propyl, butyl or pentyl group is preferable, and for a combination of R¹ and R², combinations of formyl and methyl, acetyl and methyl, propionyl and methyl, butyryl and methyl, formyl and ethyl, acetyl and ethyl, propionyl and ethyl, and butyryl and ethyl can be indicated as preferable examples.

The ALAs only need to act as an active component in the state of a 5-ALA or a derivative thereof in a living body, and can be administered as any of various salts for solubility enhancement, an ester or a prodrug (precursor) that is to be decomposed by an enzyme in the living body, depending on the form of the administration. Examples of salts of the 5-ALA and the derivative thereof can include an acid addition salt, a metal salt, an ammonium salt and an organic amine addition salt that are pharmacologically acceptable. For the acid addition salt, inorganic acid salts such as hydrochloride, hydrobromate, hydroiodide, phosphate, nitrate and sulfate, and organic acid addition salts such as formate, acetate, propionate, toluenesulfonate, succinate, oxalate, lactate, tartrate, glycolate, methanesulfonate, butyrate, valerate, citrate, fumarate, maleate and malate can be indicated as examples. For the metal salt, alkali metal salts such as a lithium salt, a sodium salt and a potassium salt, alkaline-earth metal salts such as magnesium and a calcium salt, and metal salts such as aluminum and zinc can be indicated as examples. For the ammonium salt, alkylammonium salts such as an ammonium salt and a tetramethylammonium salt can be indicated as examples. For the organic amine salt, salts such as a triethylamine salt, a piperidine salt, a morpholine salt and a toluidine salt can be indicated as examples. Here, these salts can be used in the form of a solution when used.

Among the above-indicated ALAs, 5-ALA and various esters such as 5-ALA methyl ester, 5-ALA ethyl ester, 5-ALA propyl ester, 5-ALA butyl ester and 5-ALA pentyl ester, and hydrochlorides, phosphates and sulfates thereof are desirable, and 5-ALA hydrochloride and 5-ALA phosphate can be indicated as particularly preferable examples.

The above-indicated ALAs can be manufactured by any of known methods such as chemical synthesis, production by microbes and production by enzymes. Also, each of the above-indicated ALAs may be in the form of a hydrate or a solvate, and any of the ALAs can solely be used or an arbitrary combination of two or more of the ALAs can be used.

For a dose of any of the ALAs, there are no limitations as long as the dose enables identification of a diseased tissue based on an amount of detected fluorescence produced from PpIX as a result of irradiating PpIX derived by the ALAs with excitation light using the diagnosis apparatus according to the present invention, and the dose can be 1 to 1000 mg, preferably 5 to 100 mg, more preferably, 10 to 30 mg, still more preferably 15 to 25 mg per kilogram of body weight in 5-ALA equivalent. Also, if the ALAs are used in the form of a solution, in order to prevent decomposition of the ALAs, it is preferable to prepare the solution, taking note of the avoidance of alkalification of the solution. Where the solution is alkalified, decomposition of the active component can be prevented by removal of the oxygen.

For a period of time from administration of a tumor detection agent to excitation light irradiation, a period of time during which sufficient PpIX is accumulated in the tumor tissue is desirable, and specific examples of the period of time can include two to twelve hours, preferably four to eight hours from the administration of 5-ALA.

For the light source unit that emits excitation light in the diagnosis apparatus according to the present invention, a known one can be used, and examples of the light source can include a violet LED and a violet semiconductor laser. Furthermore, in order to accurately determine whether or not one or more minute low-invasion sites scattered around a tumor part, which are difficult to observe by the naked eye is present and identify the low-invasion sites, a semiconductor laser light source having a high irradiance and a small irradiation area is preferable, and examples of an element used for the light source can include an InGaN laser diode.

Where the photosensitizing substance is PpIX, for the excitation light, light having wavelengths enabling production of fluorescence exhibiting a peak at around 636 nm, which is specific to PpIX, can be selected, furthermore, it is preferable to select light having a peak enabling production of fluorescence exhibiting a second peak at around 705 nm, which is specific to PpIX, and it is more preferable to select light having violet wavelengths close to those of ultraviolet light falling within an absorption peak for PpIX that falls within what is called the Soret band, and more specifically, the wavelengths can be wavelengths having a peak of 410 ± 10 nm, preferably 410 ± 5 nm, more preferably 410 ± 2 nm, and proper excitation light can be provided by, for example, changing a blend ratio of InGaN.

The excitation light emitted from the light source unit is guided to the excitation light irradiation/fluorescence detection optical fiber. The collimator is connected to the output/input end of the excitation light irradiation/fluorescence detection optical fiber, and the excitation light output from the output/input end is provided to a diseased tissue in such a manner that the excitation light is collimated by the collimator and excites the photosensitizing substance accumulated in the diseased tissue. Fluorescence produced by the photosensitizing substance excited by the excitation light is input from the output/input end via the same collimator, and the input fluorescence is guided to the excitation light irradiation/fluorescence detection optical fiber, and the fluorescence produced from the photosensitizing substance is detected in the detection unit.

The excitation light irradiation/fluorescence detection optical fiber may be either a single-mode optical fiber or a multi-mode optical fiber, and an optical fiber of quartz is preferable, and a core diameter of the optical fiber is preferably 50 to 1000 µm, particularly preferably 300 to 400 µm. Also, as necessary, a hollow narrow tube of plastic with the excitation light irradiation/fluorescence detection optical fiber inserted therethrough can be used.

For the collimator, e.g., a collimator-equipped connector and a SELFOC (registered trademark) lens, which are commercially available, can be indicated as examples. In order to form the excitation light output from the output/input end into a perfect parallel light beam and also accurately receive the fluorescence produced from the photosensitizing substance, it is preferable to provide a collimator. For the collimator, e.g., a glass spherical convex lens, a glass aspherical convex lens, a plastic spherical convex lens, a plastic aspherical convex lens, a quartz spherical convex lens, a quartz aspherical convex lens, a SELFOC (registered trademark) lens or a ball lens can be used; however, a quartz aspherical convex lens is preferable. A collimator having an aperture of normally 1 to 30 mm, in particular, 2 to 24 mm, is used, and a transparent tube is joined to a distal end of the collimator, enabling reliable prevention of contact between the collimator distal end and a diseased tissue, and maintenance of a fixed distance between the distal end and the tissue, whereby fluorescence spectrum data can be obtained under the same conditions. It is also possible to use a transparent rod instead of the transparent tube, bring a distal end of the rod into close contact with a tissue covered by, e.g., blood to push the blood out of the way, and measure a fluorescence spectrum. Furthermore, cutting and/or grinding the distal end of the rod at an angle of 30° to 60°, preferably 45°, enables detection of fluorescence on a side of the distal end portion. For such commercially-available collimator-equipped connector, e.g., F220SMA-A and F240APC-A manufactured by Thorlabs, Inc. can be indicated as specific examples.

On the other hand, as illustrated in Figure 4, a large-aperture collimator having a lens aperture of 20 to 30 mm is used in a state in which the collimator is nearly or actually in contact with a tissue, enabling efficient detection of red fluorescence produced by a lymph node metastasis in a human, which is often covered by fat, the red fluorescence being scattered in the fat. For such commercially-available collimator-equipped connector, e.g., F810SMA-543 manufactured by Thorlabs, Inc. can be indicated as a specific example. In Figure 4, reference numeral 31 denotes a large-aperture collimator, reference numeral 32 denotes a layer of fat, reference numeral 33 denotes a lymph node to which tumor cells spread, and reference numeral 34 denotes fluorescence scattered in the layer of fat and the tissue.

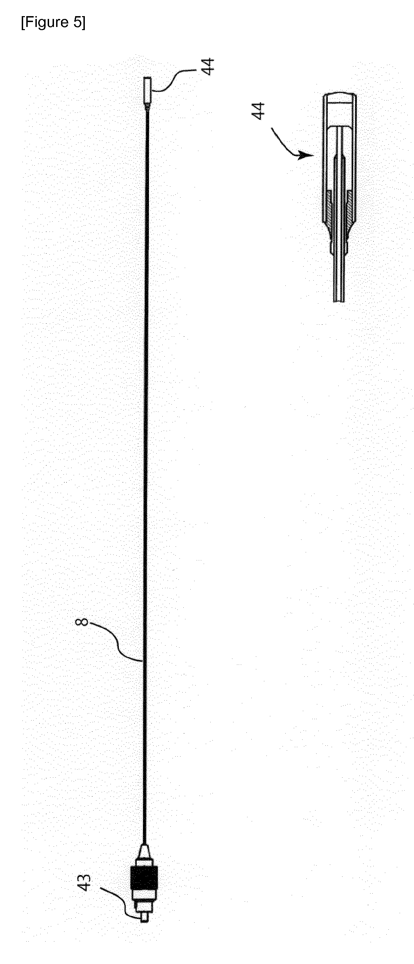

Use of an optical fiber including a distal end portion with a small-aperture collimator 44 attached thereto so that the distal end portion can be inserted into, e.g., a forceps port or a work port in a procedure using, e.g., an endoscope, a laparoscope or a thoracoscope for examination of the inside of a hollow organ, enables fluorescence excitation/detection under an endoscope, a laparoscope or a thoracoscope by inserting the optical fiber through a forceps channel of the endoscope or the work port of the laparoscope or the thoracoscope. Figure 5 illustrates an excitation light irradiation/fluorescence detection optical fiber 8 including such a distal end portion with a small-aperture collimator 44 attached thereto. An FC connector 43 is attached to the proximal end portion side of the optical fiber having a core diameter of 50 to 1000 µm, for example, 300 µm, and the FC connector 43 can be connected to a photodynamic diagnosis apparatus, providing a structure in which excitation light input from the proximal end portion side is sent to the distal end side and provided to a diseased tissue after passage through the small-aperture collimator and fluorescence produced from the diseased tissue is guided to the optical fiber 8 via the small-aperture collimator 44.

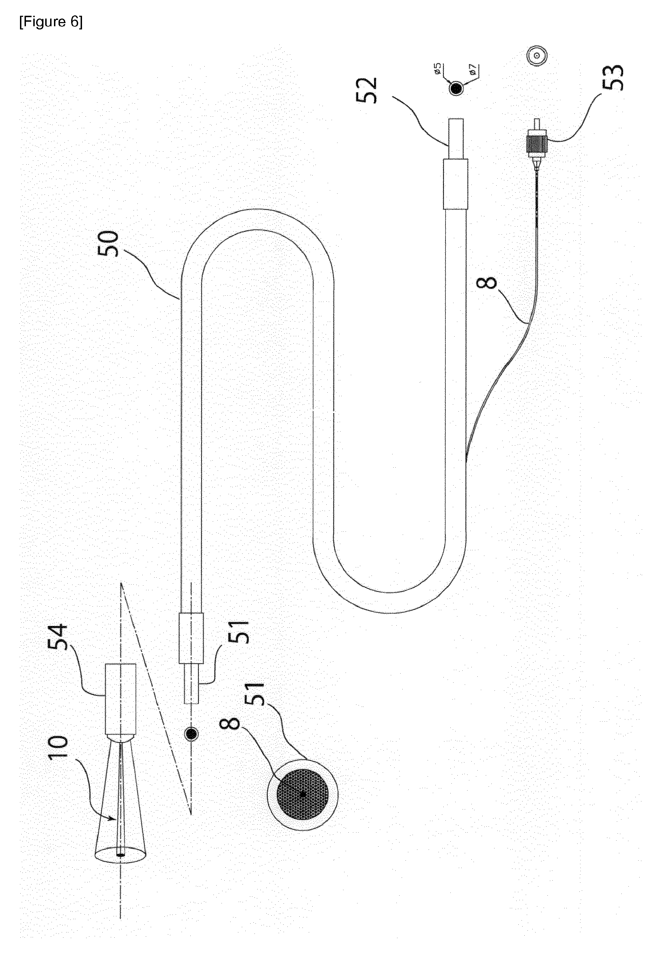

Furthermore, a hybrid fiber can be provided by incorporating an excitation light irradiation/fluorescence detection optical fiber in the center of a bundle fiber light guide that guides violet light from, e.g., a lamp light source configured to be capable of emitting violet light or a violet LED light source. For example, Figure 6 illustrates a hybrid fiber in which a fiber that guides violet laser light is incorporated in the center of a bundle fiber light guide that guides violet light from, e.g., a lamp light source configured to be capable of emitting violet light by means of insertion of a band-pass filter or a violet LED light source. In Figure 6, reference numeral 50 denotes a hybrid fiber, reference numeral 51 denotes a bundle fiber light guide, and reference numeral 8 denotes an excitation light irradiation/fluorescence detection optical fiber having a core diameter of 300 µm. Reference numeral 52 denotes a connector for connection to the violet-selectable lamp light source or the violet LED light source, the connector being connected to a connection port of the light source, and reference numeral 53 denotes a connector for connection to the diagnosis apparatus according to the present invention, the connector being connected to a connection port of the diagnosis apparatus according to the present invention (connection adapter (2) 7). Reference numeral 54 denotes a collimator unit. As described above, use of the hybrid fiber 50 with the excitation light irradiation/fluorescence detection optical fiber 8 incorporated therein enables irradiating a diseased tissue widely with violet light from the lamp light source or the LED light source to perform macroscopic fluorescence observation, and concurrently irradiating a center part of the diseased tissue with collimated violet laser light and detecting fluorescence produced from the diseased tissue in a spot. Also, for simplicity of the structure, in the hybrid fiber illustrated in Figure 6, a common collimator unit is used; however, because of the difference in NA (numerical aperture), the light from the lamp light source or the LED light source can provide a relatively wide range of irradiation and the violet laser light can be provided in the form of a relatively small spot. However, for optimization of respective light collection degrees, a separate collimator may be attached to a distal end of the fiber on the violet laser side.

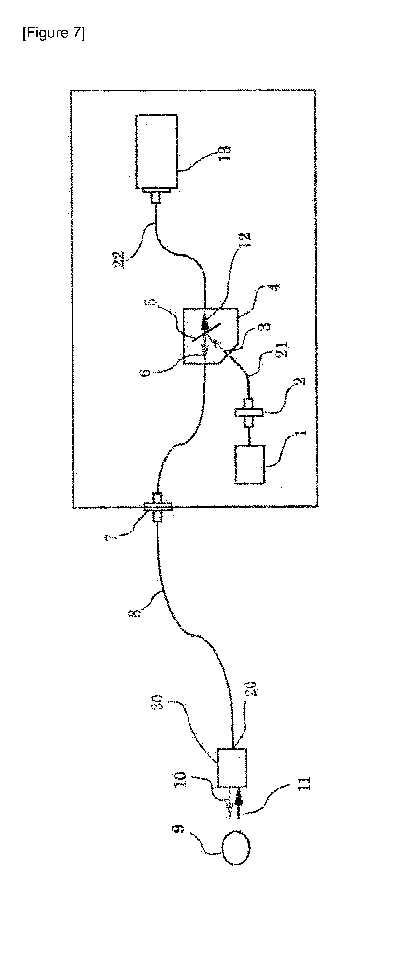

Next, a mode of a diagnosis apparatus according to the present invention including an excitation light irradiation/fluorescence detection optical fiber including an output/input end with a collimator connected thereto, and a light mixing/isolating three-port module will be described with reference to Figure 7.

Excitation light emitted from a light source unit 1 is guided to a reflection port 3 in a light mixing/isolating three-port module 4 by a light output optical fiber 21 optically connected to the light source unit 1 at a light emission end of the light source unit 1 via a connection adapter (1) 2. The excitation light guided to the reflection port 3 is reflected by a band reflection/transmission mirror 5 disposed in the light mixing/isolating three-port module 4 and is output to an excitation light irradiation/fluorescence detection optical fiber 8 from a common port 6 via a connection adapter (2) 7. The excitation light guided to the excitation light irradiation/fluorescence detection optical fiber 8 irradiates a diseased tissue 9 and the periphery thereof in the form of a parallel light beam from an output/input end 20 via a collimator 30.

The excitation light 10 output from the output/input end 20 is provided in a state in which the excitation light 10 is collimated by the collimator 30, and thereby excites a photosensitizing substance accumulated in the diseased tissue 9. Fluorescence produced by the excited photosensitizing substance is input to the excitation light irradiation/fluorescence detection optical fiber 8 from the output/input end 20 via the collimator 30, and the input fluorescence is guided from the excitation light irradiation/fluorescence detection optical fiber 8 to the common port 6 via the connection adapter (2) 7. In the light guided from the common port 6 (the fluorescence and return light of the excitation light), the return light of the excitation light is reflected by the band reflection/transmission mirror 5, and the fluorescence, which has longer wavelengths, passes through the band reflection/transmission mirror 5, and is guided to a detection unit 13 including a spectrometer by a light input optical fiber 22 via a transmission port 12. As described above, the fluorescence guided free from influence of the excitation light can be detected in the detection unit 13.

The diagnosis apparatus according to the present invention can further include spectrometric means, and for the spectrometric means, there are no specific limitations as long as the spectrometric means is, for example, one that can detect a fluorescence intensity with wavelengths of 636 ± 2 nm, preferably 636 ± 1 nm, or a fluorescence intensity with wavelengths of 626 ± 2 nm, preferably 626 ± 1 nm in addition to the above in order to detect fluorescence from PpIX; however, one that can detect fluorescence with wavelengths of 670 ± 2 nm from PpIX whose ring is partially opened is preferable from the perspective of enhancement of the accuracy of recognition of PpIX presence. More specifically, a wavelength-dispersive spectrometer such as a grating spectrometer or a prism spectrometer can be used, and such spectrometric means may be provided on an optical path from the output/input end to the detection unit or may be attached to the detection unit.

The detection unit can include signaling means for signaling based on a result of detection of a spectrum obtained by the spectrometric detection means. For the signaling means, alarm sounding means and visual signaling means are indicated as examples, and examples of the alarm sounding means can include alarm sounding means for sounding an alarm by outputting a detection sound for notification. Although, for example, a detection sound can be output if an intensity of a peak wavelength of red fluorescence, which is specific to PpIX, that is, a wavelength of 636 ± 5 nm, preferably a wavelength of 636 ± 3 nm, more preferably a wavelength of 636 ± 1 nm, is equal to or exceeds a set value or if an intensity of a wavelength of 670 ± 2 nm is equal to or exceeds a set value, in order to eliminate the influence of wavelengths of auto-fluorescence produced from a fluorescent dye in a diseased tissue and more accurately detect a peak of PpIX, it is preferable to provide alarm sounding means for, if intensities of fluorescence with a wavelength of 636 + 1 nm and a wavelength of 626 ± 1 nm are detected in the measuring device and a difference between the intensities of the fluorescence with a wavelength of 636 ± 1 nm and the fluorescence with a wavelength of 626 ± 1 nm is equal to or exceeds a set value, outputting a detection sound for notification.

Settings for changing a magnitude, an intermittent cycle and a frequency of a detection sound can be made to notify a practitioner of, for example, not only existence or non-existence of cancer cells, but also information such as the size and density of the cancer tissue. For example, it is possible that: (a) no detection sound is output for a normal cell part; (b) if the number of cancer cells is from 1 to 7 and it is determined that there is no metastasis because of the fluorescence intensity difference falling within a predetermined set value range, a detection sound of a normal volume and a relatively-long intermittent cycle is output; and (c) if the number of cancer cells is 8 or more and the fluorescence intensity difference is equal to or exceeds the predetermined set value and there may be a metastasis, a detection sound of a somewhat large volume or a somewhat short intermittent cycle is output. Use of the apparatus that allows setting of such a detection sound enables a practitioner to determine whether or not it is proper to remove a sentinel lymph node before surgery to remove a cancer, and also to confirm whether or not there is a future possibility of recurrence.

The light source unit can emit light for providing PDT on a diseased tissue, and can include a light source that, if the photosensitizing substance is PpIX, can emit light of, more specifically, wavelengths of 625 to 640 nm, preferably red light with a wavelength of 636 nm. For the light source that emits the red light, a known one can be used, and examples of the light source can include a red semiconductor laser, a red LED and a discharge lamp having an intense red light emission spectrum. The red light is guided to the excitation light irradiation/fluorescence detection optical fiber and excites PpIX specifically accumulated in a diseased tissue and thereby photoexcites peripheral oxygen molecules, and singlet oxygen generated as a result of the photoexcitation provides a cell killing effect for cancer cells by means of its strong oxidation power. Using the diagnosis apparatus according to the present invention, PDT is concurrently performed after recognition of a diseased site, further reducing the burden on the patient.

As another mode, the detection unit in the present invention can also include an image unit that displays a spectral image based on a result of detection of a spectrum obtained by the spectrometer in the spectrometric detection means. Examples of the image unit can include a diseased tissue image obtained by receiving optical signals guided by an optical fiber that doubles as an image guide fiber by means of a CCD camera and sequentially reading the optical signals as image signals corresponding to a subject image, in addition to a fluorescence spectrum peak monitoring screen. Furthermore, a substantially-colorless sharp cut filter is provided as excitation light cutting means to use the sharp cut filter, enabling removal of reflected light having short wavelengths close to ultraviolet light.

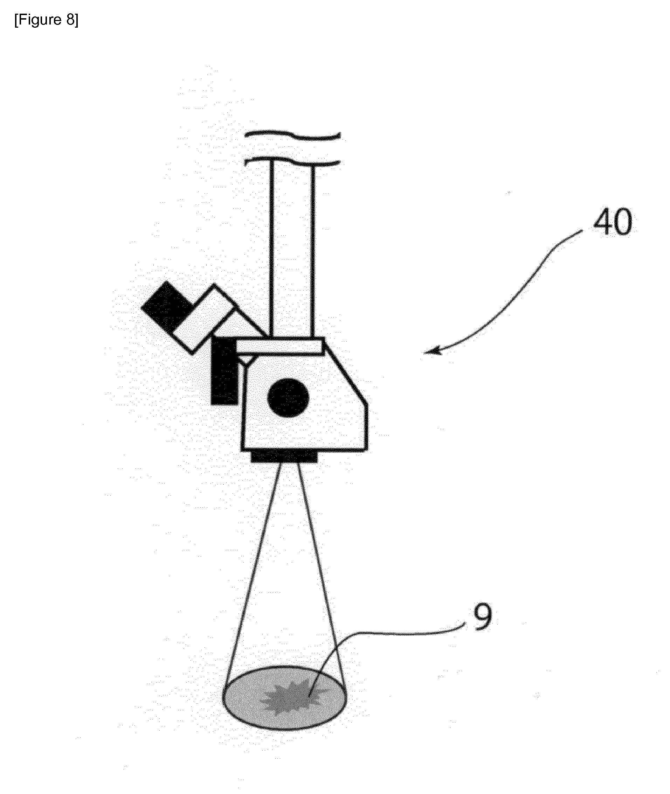

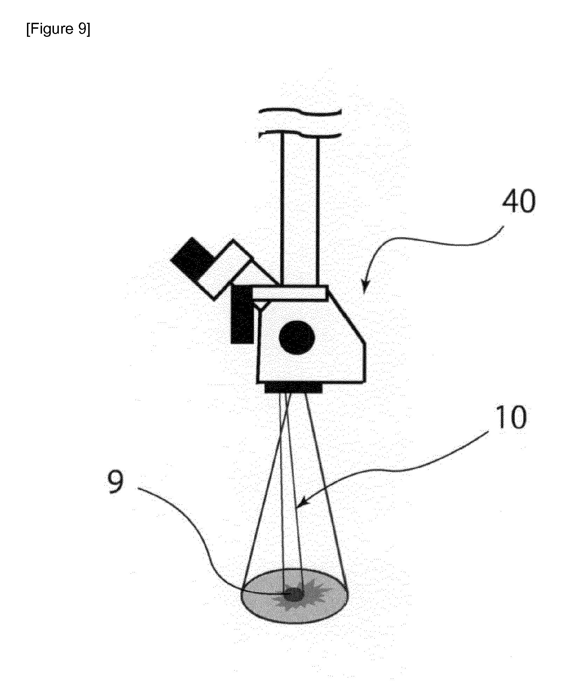

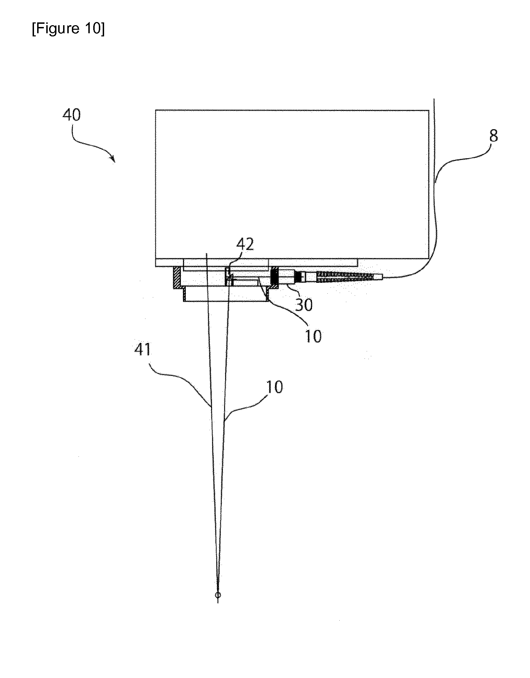

Also, an intraoperative diagnosis system according to the present invention includes a combination of a surgical microscope 40 having a fluorescence diagnosis mode, which is illustrated in Figure 8, and a diagnosis apparatus according to the present invention, and an excitation light irradiation/fluorescence detection optical fiber with a collimator connected thereto in the diagnosis apparatus is incorporated in the surgical microscope as a probe. For the incorporation as a probe, the excitation light irradiation/fluorescence detection optical fiber with the collimator connected thereto may be externally attached to the surgical microscope having a fluorescence diagnosis mode as illustrated in Figures 9 and 10; however, it is also possible that an excitation light irradiation/fluorescence detection optical fiber with a collimator connected thereto is internally provided in advance. For the surgical microscope having the fluorescence diagnosis mode, "OPMI (registered trademark) Pentero" manufactured by Carl Zeiss can be indicated as an example.

Figure 10 mentioned above illustrates a surgical microscope 40 with an excitation light irradiation/fluorescence detection optical fiber 8 externally attached thereto, a collimator 30 being connected to the excitation light irradiation/fluorescence detection optical fiber 8. The surgical microscope 40 has a structure in which in order to minimize an angle between a view direction (observation direction) 41 of the microscope and collimated excitation light 10, a prism 42 is set in a position that is as close to an objective lens as possible, excitation light is input to the prism 42 from the collimator 30 on a side and is provided downward toward the center of the field of view of the microscope, and fluorescence produced from a diseased tissue is guided to the optical fiber 8 by the collimator 30 via the prism 42. In this way, the view direction 41 of the surgical microscope and the collimated excitation light 10 are nearly coaxial to each other, which enables excitation/detection of fluorescence in a bottom part of a resection cavity in, e.g., a key hole surgery in which deep resection is performed with a small resection diameter.

### Example 1

### 1. Configuration

Specifications of an intraoperative diagnosis system including a combination of the diagnosis apparatus according to the present invention illustrated in Figure 7 and a surgical microscope used for brain tumor removal surgery are indicated below.
Detection method: sound in PpIX detection for detecting a peak of a spectrum of PpIX fluorescence derived from a violet laser diode; the pulse sound interval is changed when the PpIX fluorescence intensity increases.
Excitation light irradiation/fluorescence detection optical fiber: single core fiber (core diameter: φ300 µm) Collimator: F220SMA-A manufactured by Thorlabs, Inc.
Laser type/wavelength: InGaN laser diode/406 nm (typical) Laser output/class: fiber output 70 mW (CW operation)/class 3B
Surgical microscope: "OPMI Pentero" manufactured by Carl Zeiss

Figure 8 is a fluorescence diagnosis mode-provided surgical microscope having a function that provides violet illuminating light to an operative field by inserting/retracting a band-pass filter to/from a Xe lamp light source for illumination. Since a part of illuminating light is cut out by the filter, no sufficient excitation intensity can be obtained, and thus, a tumor body that intrinsically emits intense red fluorescence can be examined visually, but it is difficult to view red light produced by a tumor that has invaded a normal tissue that produces white fluorescence having a peak in green because a spectral sensitivity of a human for red light is 1/2 to 1/10 or lower of that for green. Furthermore, since white fluorescence produced by a normal tissue also contains red light components, it is difficult to distinguish between the both even through an observation using a band-pass filter for a red region.

Figure 9 is a schematic diagram of an intraoperative diagnosis system according to the present invention in which a collimator-equipped excitation light irradiation/fluorescence detection optical fiber is incorporated in the vicinity of an objective lens in a surgical microscope as a probe. Excitation light collimated by a collimator excites a diseased tissue at a relatively-high intensity in a small spot, and visual fluorescence also becomes intense. Concurrently, red fluorescence produced from the irradiation spot is efficiently guided to a sensor (spectrometer) by the collimator-equipped excitation light irradiation/fluorescence detection optical fiber according to the principle of telescope, whereby the fluorescence can be detected at a high sensitivity. Furthermore, since a range from which fluorescence is detected is limited to an area around the spot, a linear array sensor in the spectrometer is not saturated when illumination in the surgical microscope is switched to normal illumination, and thus, a threshold value for luminance that enables PpIX detection is enhanced.

### 2. Work and operation

An intraoperative diagnosis system in which an irradiation spot of a collimator-equipped excitation light irradiation/fluorescence detection optical fiber is connected to a PpIX-specific fluorescence detection device is set so as to align with the center of the field of view of a surgical microscope, which enables diagnosis of whether or not PpIX (substantially equal to a tumor) is localized at a site where the irradiation spot is positioned to be made easily without cumbersome work of setting a handpiece probe for fluorescence detection, providing irradiation with the probe brought close to a diseased tissue and cleaning an end face each time the end face gets dirty. As a result, it turns out that use of the present system in an actual brain tumor operation provides substantial enhancement in rate of removal of brain tumors including peripheral parts of tumor parts, and low-invasion sites around tumor parts, which are difficult to observe by the naked eye, which leads to extension of the life of a patient and amelioration of prognosis.

### Industrial Applicability

A diagnosis apparatus according to the present invention is effectively used in medical field including tumor diagnosis.

### Explanation of Letters or Numerals

- 1: light source unit
- 2: connection adapter (1)
- 3: reflection port
- 4: light mixing/isolating three-port module
- 5: band reflection/transmission mirror
- 6: common port
- 7: connection adapter (2)
- 8: excitation light irradiation/fluorescence detection optical fiber
- 81: optical fiber distal end
- 9: diseased tissue (focal site)
- 10: excitation light
- 11: fluorescence
- 12: transmission port
- 13: detection unit (spectrometer)
- 20: output/input end
- 21: light output optical fiber
- 22: light input optical fiber
- 30: collimator
- 31: large-aperture collimator
- 32: layer of fat
- 33: involved lymph node
- 34: fluorescence scattered in layer of fat or tissue
- 40: surgical microscope
- 41: view direction of microscope
- 42: prism
- 43: FC connector
- 44: small-aperture collimator
- 50: hybrid fiber
- 51: bundle fiber light guide
- 52: connector for connection to violet-selectable lamp light source or violet LED light source
- 53: connector for connection to violet laser light
- 54: collimator unit

## Claims

1. A diagnosis apparatus for identifying a diseased tissue by irradiating a photosensitizing substance accumulated in the diseased tissue with excitation light and detecting fluorescence generated from the photosensitizing substance, the apparatus comprising:
a light source unit that emits the excitation light;
a detection unit that detects the fluorescence generated from the photosensitizing substance; and
an excitation light irradiation/fluorescence detection optical fiber including an output/input end with a collimator connected thereto,
wherein the excitation light emitted from the light source unit is guided to the excitation light irradiation/fluorescence detection optical fiber, and the excitation light output from the output/input end is provided in such a manner that the excitation light is collimated by the collimator and excites the photosensitizing substance accumulated in the diseased tissue; and
the fluorescence generated by the photosensitizing substance excited by the excitation light is input from the output/input end via the collimator, and the input fluorescence is guided to the excitation light irradiation/fluorescence detection optical fiber and the fluorescence generated from the photosensitizing substance is detected in the detection unit.

2. The diagnosis apparatus according to claim 1, wherein the diseased tissue is a peripheral part of a tumor part, and/or a low-invasion site around the tumor part, the low-invasion site being difficult to observe by a naked eye.

3. The diagnosis apparatus according to claim 1 or 2, wherein the photosensitizing substance is protoporphyrin IX (PpIX).

4. The diagnosis apparatus according to any one of claims 1 to 3, wherein the excitation light irradiation/fluorescence detection optical fiber has a core diameter of 300 to 400 µm.

5. The diagnosis apparatus according to any one of claims 1 to 4, wherein the excitation light irradiation/fluorescence detection optical fiber is incorporated in a center of a bundle fiber light guide that guides violet light.

6. The diagnosis apparatus according to any one of claims 1 to 5, further comprising: a light output optical fiber optically connected to the light source unit at a light emission end of the light source unit, the light output optical fiber guiding the excitation light emitted from the light source unit to a reflection port in a light mixing/isolating three-port module; a band reflection/transmission mirror in the light mixing/isolating three-port module, the band reflection/transmission mirror reflecting the excitation light output from the reflection port, outputting the excitation light to a common port that is in communication with the excitation light irradiation/fluorescence detection optical fiber and transmitting the fluorescence input from the excitation light irradiation/fluorescence detection optical fiber to the common port; and a light input optical fiber optically connected to the detection unit at a light entrance end of the detection unit, the light input optical fiber guiding the fluorescence passing through the band reflection/transmission mirror to the detection unit via a transmission port in the light mixing/isolating three-port module.

7. The diagnosis apparatus according to any one of claims 1 to 6, further comprising a means for dispersing the fluorescence, and further comprising an alarm sounding means for, if a detected amount of the fluorescence generated from the photosensitizing substance is equal to or exceeds a set value, outputting a detection sound for notification.

8. The diagnosis apparatus according to any one of claims 1 to 7, wherein a transparent tube or a transparent rod is further joined to a distal end of the collimator.

9. An intraoperative diagnosis system comprising a combination of a diagnosis apparatus according to any one of claims 1 to 8 and a surgical microscope having a fluorescence diagnosis mode, wherein the excitation light irradiation/fluorescence detection optical fiber with the collimator connected thereto in the diagnosis apparatus is incorporated in the surgical microscope as a probe.
